# EUROPEAN PATENT APPLICATION

(11) **EP 4 586 341 A1**
(43) Date of publication of application: **16.07.2025**
(21) Application number: 24814650.8
(22) Date of filing: 19.07.2024
(51) Int. Cl.: H01M 4/583

(54) **CARBON FLUORIDE MODIFICATION METHOD, MODIFIED CARBON FLUORIDE AND LITHIUM/CARBON FLUORIDE BATTERY**

(30) Priority: 09.11.2023 CN 202311489337
(71) Applicant: Eve Energy Co., Ltd., Huizhou, Guangdong 516006 (CN)
(72) Inventor: LI, Zhiguo, Guangdong 516006 (CN)
(74) Representative: Tiburzi, Andrea
(86) International application number: PCT/CN2024/106398
(87) International publication number: WO 2024/245458

(57) **Abstract**

Provided are a carbon fluoride modification method, modified carbon fluoride and a lithium/carbon fluoride battery. The modification method comprises: mixing carbon fluoride with a composite solvent, subjecting same to a modification treatment, and then performing solid-liquid separation, so as to obtain modified carbon fluoride, wherein the composite solvent comprises water and an ether solvent. The composite solvent is used to modify carbon fluoride; and under the synergistic effect of water and the ether solvent, unstable components in a carbon fluoride material can be removed by means of reaction, thereby improving the chemical stability of the carbon fluoride material and reducing side reactions of carbon fluoride with an electrolyte solution, and therefore the storage stability of a lithium/carbon fluoride battery is improved. The modification method is simple and feasible, does not need the step of pre-charging, and is easy to implement and suitable for large-scale production.

## Description

This application claims priority to Chinese Patent Application No. 2023114893379 filed with the Chinese Patent Office on November 9, 2023, and the disclosure of which is incorporated herein by reference in its entirety.

### TECHNICAL FIELD

The present application relates to the technical field of batteries, and specifically relates to a modification method for fluorocarbon, a modified fluorocarbon, and a lithium/fluorocarbon battery.

### BACKGROUND

At present, the lithium/fluorocarbon battery has the highest energy density in lithium primary batteries. Compared with other lithium primary batteries, the lithium/fluorocarbon primary battery has the advantages of stable discharge, safe storage, and low self-discharge rate. Therefore, the lithium/fluorocarbon primary battery can be widely used in production and living, and has a wide application scenario in medical devices and military weaponry. Moreover, this battery has the characteristics of environmental protection and non-pollution, the recycling of the battery is more convenient, thus reducing the pollution to the environment, and it is an environmentally friendly battery.

However, fluorocarbon, the positive active substance in the lithium/fluorocarbon battery, is reactive with the electrolyte, especially with the organic solvent in the electrolyte, which directly affects the storage stability of the battery. To overcome the above defect, CN112993289A discloses a lithium/fluorocarbon battery, an electrolyte thereof, and a method of using the same. In the method, cyclic phosphate added to the electrolyte can form a passivation layer at the positive electrode to prevent the electrolyte from directly contacting with the fluorocarbon, and the phosphate can be decomposed to generate phosphorus-oxygen radicals and quench hydrogen radicals in the electrolyte so as to reduce the side reaction between the electrolyte and the electrode and enhance the storage performance of the battery.

CN114628670A discloses an application of nitrogen-doped/carbon-coated fluorocarbon in lithium/fluorocarbon batteries. Using an aqueous solution of trimethylolaminomethane as a buffer solution, the self-polymerization reaction of dopamine hydrochloride is firstly performed to obtain the polydopamine fluorocarbon composite material, and the composite material is calcined in an inert atmosphere to obtain the nitrogen-doped/carbon-coated fluorocarbon electrode material, wherein the carbon coating on the surface is used to prevent the electrolyte from directly contacting with the fluorocarbon to reduce the self-discharge of the battery, thus improving the storage performance of the battery.

### SUMMARY

### TECHNICAL PROBLEM

The above technique has the following shortcomings: (1) the oxidation potential of phosphate additives is higher than the open-circuit voltage of the lithium/fluorocarbon battery, it is necessary to pre-charge the battery to form a passivation layer of cyclic phosphate on the positive electrode, and this charging process may have an impact on the safety performance of the battery, which may produce lithium dendrites and puncture the separator; (2) the preparation process of the fluorocarbon coating is complicated and costly, which is not conducive to large-scale production; (3) the side reaction of the above two technical methods between the positive electrode and electrolyte is slowed down by blocking the direct contact of the positive electrode with the electrolyte, but the side reaction between the fluorocarbon and the electrolyte is not fundamentally reduced.

Therefore, it is urgent to provide a method that can fundamentally reduce the side reaction between the fluorocarbon and the electrolyte, does not require a pre-charging step, and is suitable for large-scale production.

Embodiments of the present application provide a modification method for fluorocarbon, a modified fluorocarbon, and a lithium/fluorocarbon battery. In the present application, the fluorocarbon is modified with a composite solvent, and under the synergistic effect of the water and the ether solvent, the unstable components in the fluorocarbon material can be effectively removed, the chemical stability of the material can be improved, and the side reaction between the fluorocarbon and the electrolyte can be reduced, so as to improve the storage stability of the lithium/fluorocarbon battery. And the modification method in the present application is simple and feasible, does not require pre-charging, is easy to practice, and is suitable for large-scale production.

### TECHNICAL SOLUTION

In a first aspect, the present application provides a modification method for fluorocarbon, and the modification method comprises:
mixing fluorocarbon and a composite solvent, performing a modification treatment, and then performing a solid-liquid separation to obtain a modified fluorocarbon, wherein the composite solvent comprises water and an ether solvent.

**In** a second aspect, the present application provides a modified fluorocarbon, and the modified fluorocarbon is prepared by the modification method according to the first aspect.

**In** a third aspect, the present application provides a lithium/fluorocarbon battery, and a positive electrode of the lithium/fluorocarbon battery comprises the modified fluorocarbon according to the second aspect.

### BENEFICIAL EFFECT

Compared to the related art, the beneficial effects of the present application are:
(1) the present application provides a modification method, and the fluorocarbon is subjected to a pretreatment with the composite solvent, wherein the water can react with and clean up the unstable components in the fluorocarbon, such as unstable C-F bonds, atomic fluorine between carbon layers, residual fluoride agents and catalysts from the fluoridation process, and active defective structures on the material surface, which can play the role of removing the unstable components in the fluorocarbon, and reduce the side reaction between the fluorocarbon and the electrolyte; the ether solvent has a good affinity for fluorocarbon, and it can wet the fluorocarbon effectively, and play a role of wetting and cleaning the material, and the ether can also react with unstable components in the fluorocarbon; therefore, under the combined effect of the water and the ether solvent, the unstable components in the fluorocarbon are effectively removed, and the chemical stability of the material is improved, which reduces the side reaction between the fluorocarbon and the electrolyte, improves the storage stability of the lithium/fluorocarbon battery, and avoids the capacity loss of the battery due to the side reaction in the storage process; and
(2) the modification method in the present application is simple and feasible, does not require pre-charging, is easy to practice, has a low cost, and is suitable for large-scale production.

Other aspects will be appreciated upon reading and understanding the accompanying drawings and detailed description.

### BRIEF DESCRIPTION OF DRAWINGS

FIG. 1 shows CV curves of the modified fluorocarbon provided in Example 1 and the unmodified fluorocarbon provided in Comparative Example 1 of the present application.
FIG. 2 shows infrared spectra of the modified fluorocarbon provided in Example 1 and the unmodified fluorocarbon provided in Comparative Example 1 of the present application.

### DETAILED DESCRIPTION

In a first aspect, the present application provides a modification method for fluorocarbon, and the modification method comprises:
mixing fluorocarbon and a composite solvent, performing a modification treatment, and then performing a solid-liquid separation to obtain a modified fluorocarbon, wherein the composite solvent comprises water and an ether solvent.

The present application provides a modification method, and the fluorocarbon is subjected to a pretreatment with the composite solvent, wherein the water can react with and clean up the unstable components in the fluorocarbon, such as unstable C-F bonds, atomic fluorine between carbon layers, residual fluoride agents and catalysts from the fluoridation process, and active defective structures on the material surface, which can play the role of removing the unstable components in the fluorocarbon, and reduce the side reaction between the fluorocarbon and the electrolyte; the ether solvent has a good affinity for fluorocarbon, and it can wet the fluorocarbon effectively, and play a role of wetting and cleaning the material, and the ether can also react with unstable components in the fluorocarbon; therefore, under the combined effect of the water and the ether solvent, the unstable components in the fluorocarbon are effectively removed, and the chemical stability of the material is improved, which reduces the side reaction between the fluorocarbon and the electrolyte, improves the storage stability of the lithium/fluorocarbon battery, and avoids the capacity loss of the battery due to the side reaction in the storage process; in addition, the modification method in the present application is simple and feasible, does not require pre-charging, is easy to practice, has a low cost, and is suitable for large-scale production.

In one embodiment, the ether solvent comprises at least one of dimethyl ether, ethylene glycol dimethyl ether, diethylene glycol dimethyl ether, and tetrahydrofuran, optionally ethylene glycol dimethyl ether.

In one embodiment, a mass ratio of the water and the ether solvent in the composite solvent is (10-90):(10-90), wherein the selectable range (10-90) of the water can be, for example, 10, 20, 30, 40, 50, 60, 70, 80, or 90, etc., and the selectable range (10-90) of the ether solvent can be, for example, 10, 20, 30, 40, 50, 60, 70, 80, or 90, etc.; however, the mass ratio is not limited to the listed values, and other unlisted values within the value range are also applicable.

In the present application, if the mass ratio of the water and the ether solvent is too low, the modification effect is poor; if the mass ratio of the water and the ether solvent is too high, because the proportion of water in the composite solvent is too large and the fluorocarbon is a hydrophobic material, the fluorocarbon cannot be wetted and in turn fails to be modified.

In one embodiment, a solid-liquid mass ratio of the fluorocarbon and the composite solvent is (1/10-1/2):1, which can be, for example, 0.1:1, 0.2:1, 0.3:1, 0.4:1, or 0.5:1, etc.; however, the solid-liquid mass ratio is not limited to the listed values, and other unlisted values within the value range are also applicable.

In the present application, if the solid-liquid ratio of the fluorocarbon and the composite solvent is too low, the resources will be wasted; if the solid-liquid ratio of the fluorocarbon and the composite solvent is too high, the treatment will be insufficient, and the unstable components in the fluorocarbon cannot be completely removed.

In one embodiment, the modification treatment is performed at a temperature of 0-90°C, which can be, for example, 0°C, 10°C, 20°C, 30°C, 40°C, 50°C, 60°C, 70°C, or 80°C, etc.; however, the temperature is not limited to the listed values, and other unlisted values within the value range are also applicable, optionally 40-60°C.

In the present application, the modification treatment is optionally performed at a temperature of 40-60°C. The high-temperature modification can improve the reaction activity of the unstable structures in the fluorocarbon, and improve the wettability of the fluorocarbon with the solvent, so that the reactions in pretreatment are more complete, which is conducive to the stability of the material structure. If the temperature of the modification treatment is too low, the wettability of the solvent and fluorocarbon is poor, and the reaction ability of the solvent and the active structures in the fluorocarbon is poor, which will cause incomplete removal of the unstable components from the fluorocarbon, and the material is still unstable; if the temperature of the modification treatment is too high, for the power-type fluorinated hard-carbon material, the carbon-fluorine bonds on the surface of fluorocarbon will be damaged, and the wettability of the fluorocarbon with electrolyte will become better, aggravating the de-intercalation of semi-ionic bond in fluorocarbon, aggravating the self-discharge of batteries, and deteriorating the storage performance.

In one embodiment, the modification treatment is performed for a period of more than 0.01 h, which can be, for example, 0.02 h, 0.05 h, 0.1 h, 0.5 h, 1 h, 2 h, 4 h, 6 h, 8 h, 10 h, 15 h, 20 h, 25 h, 30 h, 40 h, 45 h, or 48 h, etc.; however, the period is not limited to the listed values, and other unlisted values within the value range are also applicable, optionally 4-48 h.

In one embodiment, the modification treatment is accompanied by agitation; the agitation has a rate of 100-1000 r/min; however, the rate is not limited to the listed values, and other unlisted values within the value range are also applicable.

As an optional technical solution of the present application, the modification method specifically comprises:
adding fluorocarbon to a composite solvent composed of water and an ether solvent, and then performing a modification treatment with agitation, and then performing a solid-liquid separation, drying and dispersing to obtain the modified fluorocarbon.

Optionally, the solid-liquid separation comprises centrifugation, filtration, or natural sedimentation.

In a second aspect, the present application provides a modified fluorocarbon, and the modified fluorocarbon is prepared by the modification method according to the first aspect.

In one embodiment, shown by the cyclic voltammetry (CV) curve, the modified fluorocarbon has 1 reduction peak in the voltage range of 2-3 V. This reduction peak is assigned to the reduction peak of the lithium-fluorocarbon reaction.

In one embodiment, compared with the unmodified fluorocarbon, in the infrared spectrum of the modified fluorocarbon, the absorption peaks of the CF₂ functional group at 1332 cm⁻¹ and the semi-ionic C-F bond at 1100-1200 cm⁻¹ are weakened, which indicates that some unstable CF₂ and semi-ionic C-F bonds are removed; and no characteristic peaks of active defective structures, such as 1697 cm⁻¹ (C=O), 1631 cm⁻¹ (C=C), 1427 cm⁻¹ (CH₂), and 877 cm⁻¹ (substituent on the benzene ring), is shown within the wavelength ranges of 877-878 cm⁻¹ or 1427-1697 cm⁻¹.

In a third aspect, the present application provides a lithium/fluorocarbon battery, and a positive electrode of the lithium/fluorocarbon battery comprises the modified fluorocarbon according to the second aspect.

In one embodiment, the lithium/fluorocarbon battery further comprises a lithium metal negative electrode, an electrolyte, and a separator.

Optionally, the modified fluorocarbon positive electrode, the lithium metal negative electrode, and the separator are assembled into a cell by stacking or winding.

In one embodiment, the electrolyte comprises an organic solvent and an electrolyte salt.

In one embodiment, the electrolyte salt comprises at least one of lithium perchlorate, lithium hexafluorophosphate, lithium tetrafluoroborate, lithium bis(trifluoromethanesulfonyl) imide, lithium bis(fluorosulfonyl) imide, and lithium trifluoromethanesulfonate.

In one embodiment, the organic solvent comprises at least one of ethylene glycol dimethyl ether, ethylene carbonate, propylene carbonate, dimethyl carbonate, diethyl carbonate, and methyl ethyl carbonate.

In one embodiment, the electrolyte has a concentration of 0.5-2 mol/L, which can be, for example, 0.5 mol/L, 0.7 mol/L, 1 mol/L, 1.5 mol/L, or 2 mol/L, etc.; however, the concentration is not limited to the listed values, and other unlisted values within the value range are also applicable.

Optionally, a preparation process of the electrolyte is as follows: in a glove box filled with argon gas, mixing organic solvent components (such as propylene carbonate and ethylene glycol dimethyl ether, etc.) to obtain a mixed solvent, and adding an electrolyte salt to the mixed solvent to obtain the electrolyte.

Optionally, after the cell is assembled, the electrolyte is injected into the cell with an injection factor of 1.0-1.6 g/Ah ( which can be, for example, 1.0 g/Ah, 1.2 g/Ah, 1.4 g/Ah, or 1.6 g/Ah, etc.), and the cell is sealed to obtain a lithium/fluorocarbon battery.

The value ranges in the present application comprise not only the above listed point values, but also any unlisted point value within the above value ranges, and for reasons of space and brevity, the specific point values comprised in the ranges will not be listed exhaustively in the present application.

The technical solutions of the present application are further described below via specific embodiments.

### Example 1

This example provides a modification method for fluorocarbon, which comprises:
the fluorocarbon material was added to a composite solvent composed of water and an ether solvent, where the ether solvent was ethylene glycol dimethyl ether, a mass ratio of the water and the ethylene glycol dimethyl ether was 50:50, and a solid-liquid mass ratio of the fluorocarbon and the composite solvent was 1:5, and then stirring was started at a rate of 500 r/min and the modification treatment was performed for 24 h at 40°C, and then solid-liquid separation was performed, and the separated product was put into a blower oven for drying, and finally the dried modified fluorocarbon lumps were dispersed with a powder disperser to obtain modified fluorocarbon powder.

### Example 2

This example provides a modification method for fluorocarbon, which comprises:
the fluorocarbon material was added to a composite solvent composed of water and an ether solvent, where the ether solvent was tetrahydrofuran, a mass ratio of the water and the tetrahydrofuran was 60:40, and a solid-liquid mass ratio of the fluorocarbon and the composite solvent was 1/3:1, and then stirring was started at a rate of 300 r/min and the modification treatment was performed for 10 h at 60°C, and then solid-liquid separation was performed, and the separated product was put into a blower oven for drying, and finally the dried modified fluorocarbon lumps were dispersed with a powder disperser to obtain modified fluorocarbon powder.

### Example 3

This example provides a modification method for fluorocarbon, which comprises:
the fluorocarbon material was added to a composite solvent composed of water and an ether solvent, where the ether solvent was diethylene glycol dimethyl ether, a mass ratio of the water and diethylene glycol dimethyl ether was 50:50, and a solid-liquid ratio of the fluorocarbon and the composite solvent was 1/5:1, and then stirring was started at a rate of 400 r/min and the modification treatment was performed for 24 h at 70°C, and then solid-liquid separation was performed, and the separated product was put into a blower oven for drying, and finally the dried modified fluorocarbon lumps were dispersed with a powder disperser to obtain modified fluorocarbon powder.

### Example 4

This example differs from Example 1 in that the ethylene glycol dimethyl ether was replaced by tetrahydrofuran by an equal mass.

The remaining parameters and operation steps are exactly same as those in Example 1.

### Example 5

This example differs from Example 1 in that the solid-liquid mass ratio of the fluorocarbon and the composite solvent was adjusted to 0.05: 1.

The remaining parameters and operation steps are exactly same as those in Example 1.

### Example 6

This example differs from Example 1 in that the solid-liquid mass ratio of the fluorocarbon and the composite solvent was adjusted to 0.55: 1.

The remaining parameters and operation steps are exactly same as those in Example 1.

### Example 7

This example differs from Example 1 in that the temperature of the modification was adjusted to -5°C.

The remaining parameters and operation steps are exactly same as those in Example 1.

### Example 8

This example differs from Example 1 in that the temperature of the modification was adjusted to 95°C.

The remaining parameters and operation steps are exactly same as those in Example 1.

### Comparative Example 1

This comparative example provides a fluorocarbon material which is same as Example 1 without the composite solvent modification treatment.

### Comparative Example 2

This comparative example differs from Example 1 in that the water in the composite solvent was omitted so that the composite solvent only contained the ether solvent (i.e., ethylene glycol dimethyl ether).

The remaining parameters and operation steps are exactly same as those in Example 1.

### Performance Test

### (1) Cyclic voltammetry (CV) test

The modified fluorocarbon of Example 1 and the unmodified fluorocarbon of Comparative Example 1 were used to manufacture button cells; the cells were stored at 55°C for 14 days, and then subjected to the CV test, and the specific test conditions are: a scanning rate of 0.05 mV/s, and scanning from the open-circuit voltage of the battery at full charge to 1 V.

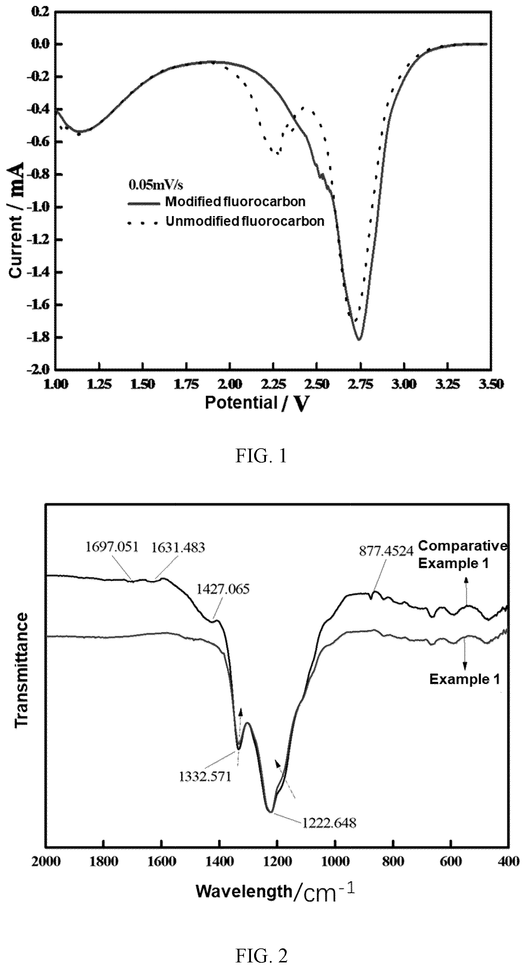

FIG. 1 shows CV curves of the unmodified fluorocarbon provided in Comparative Example 1 and the modified fluorocarbon provided in Example 1. As can be seen from FIG. 1, the unmodified fluorocarbon material has two reduction peaks in the CV curve, the reduction peak at 2.3 V is the reduction peak of the side reaction between fluorocarbon and electrolyte, which proves that the fluorocarbon material is unstable and easily has a side reaction with the electrolyte, and the deterioration of electrolyte causes a decrease in the reduction peak current of lithium-fluorocarbon reaction, the reduction peak of lithium-fluorocarbon reaction shifts to the left, and the polarization of the battery discharge process is increased; the fluorocarbon material is stabilized by the modification treatment, and the side reaction between the fluorocarbon material and the electrolyte is basically obviated, the reduction peak of the side reaction disappears, and the storage stability of the battery is improved.

### (2) Infrared spectrum test

The modified fluorocarbon in Example 1 and the unmodified fluorocarbon material in Comparative Example 1 were subjected to the infrared spectrum test in a tested range of 4000-400 cm⁻¹, as shown in FIG. 2. The test results show that after the pretreatment, the characteristic absorption peaks of the fluorocarbon material at 1697 cm⁻¹ (C=O), 1631 cm⁻¹ (C=C), 1427 cm⁻¹ (CH₂), and 877 cm⁻¹ (substituent on the benzene ring) disappear, and the characteristic absorption peaks of the fluorocarbon material at 1332 cm⁻¹ (CF₂) and 1100-1200 cm⁻¹ (the semi-ionic C-F bond) are weakened, which indicates that the unmodified fluorocarbon material has many defective structures and some carbon-fluorine bonds are unstable, and the unstable components in the material can be removed by pretreatment to enhance the stability of the material, thus enhancing the storage stability of the battery.

### (3) Storage Stability Test

The fluorocarbon materials finally provided by the above examples and comparative examples were used as the active material, separately mixed with the conductive agent Super P and the binder polyvinylidene fluoride (PVDF), and prepared into a high-viscosity slurry with a viscosity of 12000 cp according to the mass ratio of the active material, the conductive agent, and the binder of 87:8:5, and the slurry was coated on the carbon-coated aluminum foil on Side A and Side B in sequence one time for each side, with a coating thickness of 100 µm and the loading capacity of the electrode sheet of 120 g/m², dried at 100°C, and subjected to roller pressing with compacted density of 1.26 g/cm³ and a porosity of 52%, and then cut or die-cut into an appropriate size to obtain a fluorocarbon positive electrode sheet; the lithium strip was cut to the appropriate size to obtain a lithium negative electrode sheet with a thickness of 50 µm; the modified fluorocarbon positive electrode sheet, the lithium metal negative electrode sheet, and the separator were assembled into a cell by stacking, and an electrolyte of the primary lithium fluorocarbon battery was injected into the cell, and the organic solvent in the electrolyte was propylene carbonate (PC) and dimethoxyethane (DME), and the electrolyte salt was lithium trifluoromethanesulfonate LiTFA and lithium perchlorate LiClO₄, wherein a concentration of the electrolyte was 1 mol/L, and the injection factor was 1.2 g/Ah, and the lithium/carbon fluoride battery was obtained after the sealing.

Test conditions: (a) at room temperature, the lithium/fluorocarbon battery was discharged at 0.01 C with a discharge cut-off voltage of 1.5 V, and the discharge capacity of the new battery was recorded; (b) after the new same lithium/fluorocarbon battery was stored at a high temperature of 55°C for 14 days, the discharge capacity of the battery was tested again under the above conditions and recorded, and the capacity retention rate was calculated, wherein the capacity retention rate = discharge capacity after the high-temperature storage / discharge capacity of the new battery * 100%.

**Table 1**

| | Capacity retention ratio (%) |
|---|---|
| Example 1 | 99.89 |
| Example 2 | 99.2 |
| Example 3 | 99.5 |
| Example 4 | 98.60 |
| Example 5 | 99.6 |
| Example 6 | 98.0 |
| Example 7 | 94.7 |
| Example 8 | 89.31 |
| Comparative Example 1 | 93.86 |
| Comparative Example 2 | 95.5 |

### Analysis

As can be seen from the results of Examples 1-3 and Comparative Example 1, in the present application, the fluorocarbon is modified with a composite solvent composed of the water and the ether solvent, and under the synergistic effect of the water and the ether solvent, the unstable components in the fluorocarbon material can be effectively removed by the reaction, the chemical stability of the material can be improved, and the side reaction between the fluorocarbon and the electrolyte can be reduced, especially the side reaction between oxidative fluorine in fluorocarbon and ethylene glycol dimethyl ether, so as to improve the storage stability of the lithium/fluorocarbon battery and maintain stable capacity. As can be seen from Table 1, in Examples 1-3, the capacity retention rate is relatively high, which indicates that the modified fluorocarbon material has a good stability, and the modified fluorocarbon battery has a reduced self-discharge and good storage stability; in Comparative Example 1, the capacity retention rate is relatively low, which indicates that the battery assembled by the unmodified fluorocarbon has a large self-discharge and poor storage stability after stored at high temperature for 14 days.

As can be seen from the results of Example 1 and Example 4, the ethylene glycol dimethyl ether and water, which serve as the composite solvent, are used for modification, and the corresponding battery has a superior capacity retention rate and better storage stability.

As can be seen from the results of Example 1 and Examples 5-6, if the solid-liquid mass ratio of the fluorocarbon and the composite solvent is too low, the resources will be wasted; if the solid-liquid ratio of the fluorocarbon and the composite solvent is too high, the treatment will be insufficient, the unstable components in the fluorocarbon will not be completely removed, and the capacity retention rate of the battery will be relatively low.

As can be seen from the results of Example 1 and Examples 7-8, if the temperature of the modification treatment is too low, the wettability of the fluorocarbon with solvent is poor, and the reaction ability of the solvent and the active structure in the fluorocarbon is poor, which will cause incomplete removal of the unstable components from the fluorocarbon, and the capacity retention rate of the battery is low; if the temperature of the modification treatment is too high, for the power-type fluorinated hard carbon material, the carbon-fluorine bonds on the surface of fluorocarbon will be damaged, and the wettability of the fluorocarbon with electrolyte will become better, aggravating the de-intercalation of semi-ionic bond in fluorocarbon, aggravating the self-discharge of batteries, and deteriorating the storage performance.

As can be seen from the results of Example 1 and Comparative Example 2, if only ether solvents are used for modification, the improvement of battery storage stability is limited, and the ether solvent cannot significantly improve the storage stability of the battery alone.

## Claims

1. A modification method for fluorocarbon, which comprises:
mixing fluorocarbon and a composite solvent, performing a modification treatment, and then performing a solid-liquid separation to obtain a modified fluorocarbon, wherein the composite solvent comprises water and an ether solvent.

2. The modification method according to claim 1, wherein the ether solvent comprises at least one of dimethyl ether, ethylene glycol dimethyl ether, diethylene glycol dimethyl ether, and tetrahydrofuran, and optionally ethylene glycol dimethyl ether.

3. The modification method according to claim 1 or 2, wherein in the composite solvent, a mass ratio of the water and the ether solvent is (10-90):(10-90).

4. The modification method according to any one of claims 1-3, wherein a solid-liquid mass ratio of the fluorocarbon and the composite solvent is (1/10-1/2): 1.

5. The modification method according to any one of claims 1-4, wherein the modification treatment is performed at a temperature of 0-90°C, and optionally 40-60°C.

6. The modification method according to any one of claims 1-5, wherein the modification treatment is performed for a period of more than 0.01 h, and optionally 4-48 h.

7. The modification method according to any one of claims 1-6, wherein the modification treatment is accompanied by agitation, and the agitation has a rate of 100-1000 r/min.

8. The modification method according to any one of claims 1-7, wherein the modification method specifically comprises:
adding fluorocarbon to a composite solvent consisting of water and an ether solvent, and performing a modification treatment with agitation, and then performing a solid-liquid separation, drying, and dispersing to obtain the modified fluorocarbon.

9. A modified fluorocarbon, which is prepared by the modification method according to any one of claims 1-8.

10. The modified fluorocarbon according to claim 9, wherein the modified fluorocarbon has 1 reduction peak in a voltage range of 2-3 V in a cyclic voltammetry curve.

11. A lithium/fluorocarbon battery, wherein a positive electrode of the lithium/fluorocarbon battery comprises the modified fluorocarbon according to claim 9 or 10.

12. The lithium/fluorocarbon battery according to claim 11, wherein the lithium/fluorocarbon battery further comprises a lithium metal negative electrode, an electrolyte, and a separator.

13. The lithium/fluorocarbon battery according to claim 12, wherein the electrolyte has a concentration of 0.5-2 mol/L.
